(19) Europäisches Patentamt
European Patent Office
Office européen des brevets

(11) Numéro de publication: **0 077 746**
**B1**

(12) # FASCICULE DE BREVET EUROPEEN

(45) Date de publication du fascicule du brevet: **12.06.85**

(51) Int. Cl.⁴: **C 07 C 51/56**, C 07 C 53/12

(21) Numéro de dépôt: **82420146.1**

(22) Date de dépôt: **19.10.82**

(54) **Procédé de préparation de l'anhydride acétique.**

(30) Priorité: **21.10.81 FR 8120007**

(43) Date de publication de la demande: **27.04.83 Bulletin 83/17**

(45) Mention de la délivrance du brevet: **12.06.85 Bulletin 85/24**

(84) Etats contractants désignés: **AT BE CH DE FR GB IT LI LU NL SE**

(56) Documents cités:
**RESEARCH DISCLOSURE, no. 208, août 1981, page 327, no. 20832, Havant Hampshire, G.B.**
**CHEMICAL REVIEWS, vol. 74, no. 3, juin 1974, pages 351-384, American Chemical Society, Michigan, USA**

(73) Titulaire: **RHONE-POULENC CHIMIE DE BASE, 25, quai Paul Doumer, F-92408 Courbevoie (FR)**

(72) Inventeur: **Gauthier-Lafaye, Jean, 21, rue Duguesclin, F-69006 Lyon (FR)**
Inventeur: **Perron, Robert, La Pécollère, F-69390 Charly (FR)**

(74) Mandataire: **Varnière-Grange, Monique et al, RHONE-POULENC RECHERCHES Service Brevets Chimie et Polymères Centre de Recherches de Saint-Fons 85, rue des Frères Perret B.P. 62, F-69192 St-Fons Cédex (FR)**

EP 0 077 746 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de l'anhydride acétique par carbonylation de l'acétate de méthyle.

La présente invention a plus particulièrement pour objet un procédé de préparation de l'anhydride acétique par réaction de l'oxyde de carbone sur l'acétate de méthyle en milieu anhydre, en phase liquide sous une pression totale comprise entre 15 et 700 bar et à une température comprise entre 140 et 300°C, en présence d'une quantité efficace de nickel, d'iodure de méthyle, d'un iodure alcalin et d'un éther-couronne.

Le procédé selon l'invention nécessite la présence d'une quantité efficace de nickel. N'importe quelle source de nickel peut être mise en œuvre dans le cadre du présent procédé. On peut introduire le nickel sous sa forme métallique (nickel Raney, par exemple) ou sous toute autre forme commode. A titre d'exemples de composés du nickel susceptibles d'être utilisés poour la mise en œuvre du présent procédé, on peut citer le carbonate, l'oxyde, l'hydroxyde, les halogénures, en particulier l'iodure, les carboxylates, en particulier l'acétate, de nickel.

Néanmoins, lorsqu'on charge un sel de nickel, on peut observer une période d'induction plus ou moins longue et, de ce fait, on peut être amené à préférer l'emploi de composés de nickel zéro tel que le nickel tétracarbonyle et le bis(triphénylphosphine)nickel dicarbonyle. Bien entendu, les spécialistes seront à même de déterminer les formes appropriées des composés du nickel et ils verront, naturellement, que la forme précise sous laquelle le nickel est introduit dans le milieu réactionel n'est pas fondamentale, en particulier dans le cadre d'un procédé continu.

La quantité de nickel n'est pas critique. La teneur en nickel qui a une influence sur la vitesse de réaction est déterminée en fonction de la vitesse de réaction que l'on estime convenable, compte tenu des autres paramètres de la réaction. De manière générale, une quantité comprise entre 5 et 2000 milliatomes-grammes de nickel par litre de solution conduit à des résultats satisfaisants. On opère de préférence avec une teneur comprise entre 20 et 1000 milliatomes-grammes de nickel par litre.

La mise en œuvre de la présente invention nécessite également la présence dans le milieu réactionnel d'iodure de méthyle. Il n'est pas nécessaire de charger au départ ce composant du système catalytique et on peut faire appel, par exemple, à de l'iode libre, à de l'acide iodhydrique, à un iodure d'alkyle, différent de l'iodure de méthyle, ou à un iodure d'acyle. Comme les spécialistes le savent, l'iode et ces types de composés iodés peuvent être considérés comme des précurseurs de l'iodure de méthyle dans la réaction en cause.

En général, l'iodure de méthyle est présent dans le milieu réactionnel à raison de 1 à 100 mol et, de préférence, à raison de 5 à 50 mol par atome-gramme de nickel se trouvant dans ledit milieu.

Le système catalytique mis en œuvre dans le cadre du présent procédé comprend également un iodure alcalin. La titulaire préconise l'emploi de l'iodure de sodium ou de potassium.

En général, l'iodure alcalin est utilisé à raison de 0,2 à 50 mol par atome-gramme de nickel présent dans le milieu réactionnel; de préférence, on utilise de 0,5 à 20 mol d'iodure alcalin par atome-gramme de nickel et, de manière avantageuse, de 1 à 10 mol par atome-gramme de nickel.

Le système catalytique mis en œuvre dans le cadre du présent procédé comprend également un éther-couronne. Ces composés sont des polyéthers macrocycliques, bien connus en eux-mêmes, qui peuvent être représentés par les formules I à III ci-après:

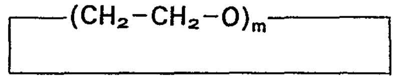 (I)

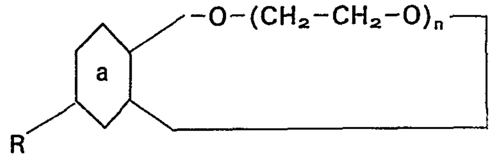 (II)

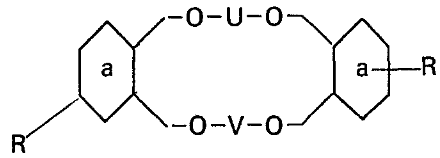 (III)

dans lesquelles:

m et n sont des nombres entiers tels que les macrocycliques renferment de 3 à 20 atomes d'oxygène,

a désigne un radical phénylène, cyclohexylène ou naphtylène-2,3,

R représente un radical alkyle ayant au maximum 4 atomes de carbone ou d'hydrogène,

U et V, identiques ou différents, représentent un radical de formule:

$$-(CH_2-CH_2-O)_{p}(CH_2)_{q} \quad (IV)$$

dans laquelle p est un nombre entier pouvant être nul et tel que le cycle renferme au maximum 20 atomes d'oxygène, et q est un nombre entier compris entre 1 et 10, q étant de préférence égal à 2 lorsque p est distinct de 0.

Pour de plus amples informations relatives notamment à la préparation des éthers-couronnes on se reportera aux références suivantes:

– «Journal of the Chemical Society», 1967, vol. 89, pp. 7017 et suivantes;

– «Chemical Reviews», 1974, vol. 74, N° 3, pp. 351 et suivantes.

A titre d'exemples d'éthers-couronnes susceptibles de convenir à la mise en œuvre de la présente invention, on peut citer:

l'éther-couronne (12-4)
l'éther-couronne (15-5)
l'éther-couronne (18-6)
l'éther-couronne (21-7
l'éther-couronne (24-8)
le benzo-couronne (9-3)
le benzo-couronne (12-4)

le benzo-couronne (15-5)
le benzo-couronne (18-6)
le (t-butylbenzo)-couronne (15-5)
le (t-butylbenzo)-couronne (18-6)
le cyclohexyl-couronne (12-4)
le cyclohexyl-couronne (15-5)
le cyclohexyl-couronne (18-6)
le (tertiobutylcyclohexyl)-couronne (15-5)
le (tertiobutylcyclohexyl)-couronne (18-6)
le (naphto-2,3)-couronne (15-5)
le (naphto-2,3)-couronne (18-6)
le dibenzo-couronne (14-4)
le dibenzo-couronne (15-5)
le dibenzo-couronne (16-5)
le dibenzo-couronne (18-6)
le dibenzo-couronne (21-7)
le dibenzo-couronne (24-8)
le dibenzo-couronne (30-10)
le dibenzo-couronne (60-20)
le di(tertiobutylbenzo)-couronne (18-6)
le di(naphto-2,3)-couronne (18-6)
le dicyclohexyl-couronne (18-6)
le di(tertiobutylcyclohexyl)-couronne(18-6)
le dicyclohexyl-couronne (24-8)
le dicyclohexyl-couronne (30-10)
le dicyclohexyl-couronne (60-20)

On fera appel, de préférence, aux polyéthers macrocycliques de formule I à III ci-avant renfermant de 5 à 10 atomes d'oxygène dans le cycle. Parmi les polyéthers de formule III, on choisira de préférence ceux dans lesquels U et V sont identiques, q est égal à 2 et p est un entier égal à 1, 2 ou 3, et R représente l'hydrogène.

L'éther-couronne (15-5) et l'éther-couronne (18-6) conviennent plus particulièrement à la mise en œuvre du présent procédé.

La quantité d'éther-couronne à mettre en œuvre peut varier dans de larges limites. Aucun avantage n'est observé lorqu'on utilise plus de 1 mol d'éther-couronne par mole d'iodure alcalin. En général, la quantité d'éther-couronne est d'au moins 1 à 5% molaire par rapport à l'iodure alcalin. La quantité optimale d'éther-couronne à mettre en œuvre dépendra pour une large mesure de la nature et de la quantité de l'iodure alcalin utilisé ainsi que de la nature de l'éther-couronne lui-même. Elle est en général de l'ordre de 10 à 75% molaire par rapport à l'iodure alcalin.

Selon une variante particulièrement avantageuse du présent procédé, le système catalytique renferme en outre un sel alcalino-terreux ou, de préférence, un sel de lithium.

La nature précise de l'anion de ce sel n'est pas fondamentale et, à titre d'exemples de sels utilisables dans le cadre du présent procédé, on peut citer: les hydroxydes, les chlorures, les bromures, les iodures, les carbonates et les nitrates ainsi que les carboxylates renfermant au maximum 12 atomes de carbone.

Parmi ces sels, l'iodure, le carbonate et les carboxylates de lithium conviennent particulièrement bien à la mise en œuvre de la présente invention. On utilise, de préférence, un carboxylate de lithium ayant au maximum 5 atomes de carbone, l'acétate de lithium se révélant particulièrement efficace.

En général, on utilise un ou plusieurs sels de lithium, de magnésium ou de calcium en une quantité telle que le rapport atomique du métal (M) au nickel soit compris entre 1 et 100, bien que des quantités inférieures ou supérieures puissent être mises en jeu. De bons résultats sont obtenus pour un rapport atomique M/Ni compris entre 2 et 25.

Le système catalytique défini ci-avant se révèle particulièrement efficace pour préparer l'anhydride acétique par carbonylation de l'acétate de méthyle en phase liquide.

Comme indiqué en tête du présent brevet, la réaction est conduite en phase liquide sous une pression supérieure à la pression atmosphérique. En général, on opère sous une pression totale supérieure à 15 bar; il n'est pas utile d'atteindre 700 bar. Pour une bonne mise en œuvre de l'invention, une pression totale de 25 à 200 bar est préconisée.

La température de réaction est généralement supérieure à 140° C, sans qu'il soit nécessaire d'atteindre 300° C. De bons résultats sont obtenus dans la gamme de températures allant de 160 à 220° C.

On met en œuvre, de préférence, du monoxyde de carbone sous forme essentiellement pure, tel qu'il se présente dans le commerce. Toutefois, la présence d'impuretés telles que du dioxyde de carbone, de l'oxygène, du méthane et de l'azote peut être tolérée. La présence d'hydrogène n'est pas nuisible, même en des proportions relativement importantes.

En fin d'opération, l'anhydride acétique obtenu est séparé des autres constituants du milieu réactionnel par tout moyen approprié, par exemple par distillation.

Les exemples ci-après illustrent l'invention sans toutefois en limiter le domaine ou l'esprit.

Dans ce qui suit, RR (%) désigne le nombre de moles d'anhydride acétique produites pour 100 mol d'acétate de méthyle chargées.

*Exemple 1:*

Dans un autoclave en Hastelloy B 2 de 125 ml de capacité, on charge 25 ml d'acétate de méthyle, 20 ml d'anhydride acétique, 8 mmol de nickel tétracarbonyle, 80 mmol d'iodure de méthyle, 100 mmol d'iodure de sodium et 20 mmol d'éther-couronne (15-5). Après fermeture de l'autoclave, on établit une pression de 40 bar de monoxyde de carbone. L'agitation par un système de va-et-vient est mise en route et l'autoclave est porté en 20 min environ à 180° C au moyen d'un four annulaire. La pression dans l'autoclave est alors de 64 bar; elle est ensuite maintenue constante et égale à 70 bar par des recharges successives de monoxyde de carbone.

Après 3 h de réaction à 180° C, l'agitation et le chauffage sont arrêtés; l'autoclave est refroidi et dégazé. Le mélange réactionnel est alors analysé. On obtient 79 g/h × l d'anhydride acétique: RR (%) = 38.

*Essai témoin a:*

On reproduit l'exemple 1 ci-avant en omettant

de charger l'éther-couronne. Après 2 h à 180° C, on n'observe aucune réaction.

*Exemple 2:*

Dans un autoclave en Hastelloy B 2 de 125 ml de capacité, on charge 25 ml d'acétate de méthyle, 20 ml d'anhydride acétique, 8 mmol de nickel tétracarbonyle, 79 mmol d'iodure de méthyle, 100 mmol d'iodure de potassium et 20 mmol d'éther-couronne (18-6). Après fermeture de l'autoclave, on établit une pression de 40 bar de monoxyde de carbone. L'agitation par un système de va-et-vient est mise en route et l'autoclave est porté en 20 min environ à 180° C au moyen d'un four annulaire. La pression dans l'autoclave est alors de 64 bar; elle est ensuite maintenue constante et égale à 70 bar par des recharges successives de monoxyde de carbone.

Après 2 h de réaction à 180° C, l'agitation et le chauffage sont arrêtés; l'autoclave est refroidi et dégazé. Le mélange réactionnel est alors analysé. On obtient 40 g/h × l d'anhydride acétique: RR (%) = 15.

*Exemple 3:*

Dans un autoclave en Hastelloy B 2 de 125 ml de capacité, on charge 25 ml d'acétate de méthyle, 20 ml d'anhydride acétique, 8 mmol de nickel tétracarbonyle, 80 mmol d'iodure de méthyle, 100 mmol d'iodure de sodium et 20 mmol d'éther-couronne (15-5) et 40 mmol d'acétate de lithium. Après fermeture de l'autoclave, on établit une pression de 40 bar de monoxyde de carbone. L'agitation par un système de va-et-vient est mise en route et l'autoclave est porté en 20 min environ à 180° C au moyen d'un four annulaire. La pression dans l'autoclave est alors de 64 bar; elle est ensuite maintenue constante et égale à 70 bar par des recharges successives de monoxyde de carbone.

Après 2 h de réaction à 180° C, l'agitation et le chauffage sont arrêtés; l'autoclave est refroidi et dégazé. Le mélange réactionnel est alors analysé. On obtient 135 g/h × l d'anhydride acétique: RR (%) = 36.

**Revendications**

1. Procédé de préparation de l'anhydride acétique par réaction de l'oxyde de carbone sur l'acétate de méthyle en milieu anhydre, en phase liquide, sous une pression totale comprise entre 15 et 700 bar et à une température comprise entre 140 et 300° C, en présence d'une quantité efficace de nickel, d'iodure de méthyle, caractérisé en ce qu'on opère également en présence simultanée d'un iodure alcalin et d'un éther-couronne.

2. Procédé selon la revendication 1, caractérisé en ce que la pression totale est comprise entre 25 et 200 bar.

3. Procédé selon l'une des revendications 1 ou 2, caractérisé en ce que la température est comprise entre 160 et 220° C.

4. Procédé selon l'une des revendications précédentes, caractérisé en ce que la concentration du nickel est comprise entre 5 et 2000 milliatomes-grammes par litre de milieu réactionnel.

5. Procédé selon la revendication 4, caractérisé en ce que la concentration du nickel est comprise entre 20 et 1000 milliatomes-grammes par litre de milieu réactionnel.

6. Procédé selon l'une des revendications précédentes, caractérisé en ce que l'éther-couronne est choisi parmi les polyéthers macrocycliques de formules (I) à (III):

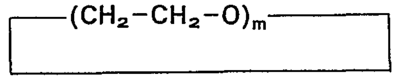
(I)

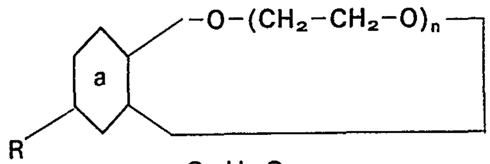
(II)

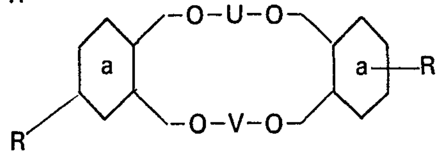
(III)

dans lesquelles:

m et n sont des nombres entiers tels que les macrocycliques renferment de 3 à 20 atomes d'oxygène,

a désigne un radical phénylène, cyclohexylène ou naphtylène-2,3,

R représente un radical alkyle ayant au maximum 4 atomes de carbone ou d'hydrogène,

U et V, identiques ou différents, représentent un radical de formule (IV):

$$-(CH_2-CH_2-O)_{\overline{p}}(CH_2)_{\overline{q}} \quad (IV)$$

dans laquelle p est un nombre entier pouvant être nul et tel que le cycle renferme au maximum 20 atomes d'oxygène, et q est un nombre entier compris entre 1 et 10, q étant, de préférence, égal à 2 lorsque p est distinct de 0.

7. Procédé selon la revendication 6, caractérisé en ce que l'éther-couronne renferme de 5 à 10 atomes d'oxygène dans le cycle.

8. Procédé selon l'une des revendications précédentes, caractérisé en ce que l'éther-couronne est choisi parmi les composés de formule (III):

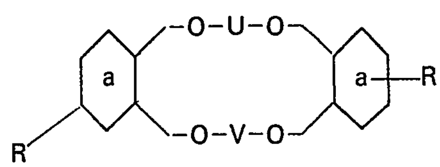
(III)

dans laquelle:

a désigne un radical phénylène, cyclohexylène ou naphtylène-2,3,

R représente l'hydrogène,

U et V, identiques, représentent un radical de formule:

$$-(CH_2-CH_2-O)_{\overline{p}}(CH_2)_2$$

dans laquelle p est égal à 1, 2 ou 3.

9. Procédé selon la revendication 1, caractérisé en ce que l'éther-couronne est choisi parmi

l'éther-couronne (15-5) et l'éther-couronne (18-6).

10. Procédé selon l'une des revendications précédentes, caractérisé en ce que l'iodure de méthyle est présent dans le milieu réactionnel à raison de 1 à 100 mol et, de préférence, de 5 à 50 mol par atome-gramme de nickel.

11. Procédé selon l'une des revendications précédentes, caractérisé en ce que l'iodure alcalin est choisi parmi l'iodure de sodium et l'iodure de potassium.

12. Procédé selon l'une des revendications précédentes, caractérisé en ce que l'iodure alcalin est utilisé à raison de 0,2 à 50 mol par atome-gramme de nickel.

13. Procédé selon la revendication 12, caractérisé en ce que l'iodure alcalin est utilisé à raison de 0,5 à 20 et, et de préférence de 1 à 10 mol par atome-gramme de nickel.

14. Procédé selon l'une des revendications précédentes, caractérisé en ce que l'éther-couronne représente au moins 5% (molaire) de l'iodure alcalin.

15. Procédé selon l'une des revendications précédentes, caractérisé en ce que l'éther-couronne représente au plus 1 mol par mole d'iodure alcalin.

16. Procédé selon l'une des revendications précédentes, caractérisé en ce que l'éther-couronne représente de 10 à 50% (molaire) de l'iodure alcalin.

17. Procédé selon l'une des revendications précédentes, caractérisé en ce que l'on opère également en présence d'un sel de lithium.

18. Procédé selon la revendication 16, caractérisé en ce que le sel de lithium est un carboxylate ayant au maximum 12 atomes de carbone.

19. Procédé selon l'une des revendications 17 ou 18, caractérisé en ce que le sel de lithium est l'acétate de lithium.

**Patentansprüche**

1. Verfahren zur Herstellung von Essigsäureanhydrid durch Umsetzung von Kohlenoxid mit Methylacetat in wasserfreiem Medium, in flüssiger Phase, unter einem Gesamtdruck von 15 bis 700 bar und bei einer Temperatur von 140 bis 300°C, in Gegenwart einer wirksamen Menge Nikkel, Methyliodid, dadurch gekennzeichnet, dass man ebenfalls bei gleichzeitiger Anwesenheit eines Alkaliiodids und eines Kronenethers arbeitet.

2. Verfahren nach Anspruch 1, dadurch gekennzeichnet, dass der Gesamtdruck 25 bis 200 bar beträgt.

3. Verfahren nach einem der Ansprüche 1 oder 2, dadurch gekennzeichnet, dass die Temperatur 160 bis 220°C beträgt.

4. Verfahren nach einem der vorangehenden Ansprüche, dadurch gekennzeichnet, dass die Nickelkonzentration 5 bis 2000 mG-Atom je Liter Reaktionsmedium ausmacht.

5. Verfahren nach Anspruch 4, dadurch gekennzeichnet, dass die Nickelkonzentration 20 bis 1000 mG-Atom je Liter Reaktionsmedium ausmacht.

6. Verfahren nach einem der vorangehenden Ansprüche, dadurch gekennzeichnet, dass der Kronenether ausgewählt wird aus den makrocyclischen Polyethern der Formeln (I) bis (III):

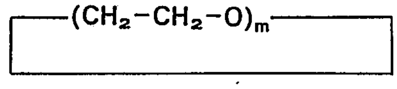

(I)

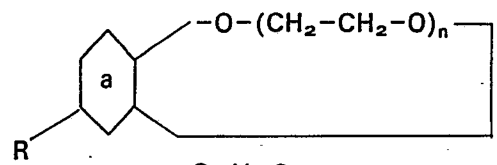

(II)

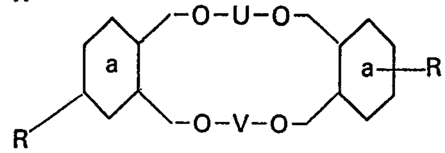

(III)

in denen:

m und n ganze Zahlen sind, derart, dass die Makrocyclen 3 bis 20 Sauerstoffatome enthalten,

a eine Phenylen-, Cyclohexylen- oder 2,3-Naphthylengruppe bedeutet,

R eine Alkylgruppe mit maximal 4 Kohlenstoffatomen oder Wasserstoff ist,

U und V gleich oder verschieden sein können und jeweils für eine Gruppe der Formel (IV):

$$-(CH_2-CH_2-O)_p(CH_2)_q \quad (IV)$$

stehen, in der p eine ganze Zahl und 0 ist, derart, dass der Cyclus maximal 20 Sauerstoffatome enthält und q eine ganze Zahl von 1 bis 10 ist, wobei q vorzugsweise 2 ist, wenn p von 0 verschieden ist.

7. Verfahren nach Anspruch 6, dadurch gekennzeichnet, dass der Kronenether 5 bis 10 Sauerstoffatome im Cyclus enthält.

8. Verfahren nach einem der vorangehenden Ansprüche, dadurch gekennzeichnet, dass der Kronenether ausgewählt wird unter den Verbindungen der Formel (III):

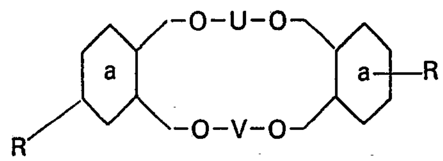

(III)

in der:

a eine Phenylen-, Cyclohexylen- oder 2,3-Naphthylengruppe bedeutet,

R für Wasserstoff steht,

U und V identisch sind und jeweils für eine Gruppe der Formel:

$$-(CH_2-CH_2-O)_p(CH_2)_2$$

stehen, in der p 1, 2 oder 3 ist.

9. Verfahren nach Anspruch 1, dadurch gekennzeichnet, dass der Kronenether augewählt wird aus [15]-Krone-5 und [18]-Krone-6.

10. Verfahren nach einem der vorangehenden Ansprüche, dadurch gekennzeichnet, dass das Methyliodid im Reaktionsmedium in einer Menge von 1 bis 100 mol, vorzugsweise von 5 bis 50 mol je Grammatom Nickel vorhanden ist.

11. Verfahren nach einem der vorangehenden

Ansprüche, dadurch gekennzeichnet, dass das Alkaliiodid aus Natriumiodid und Kaliumiodid ausgewählt wird.

12. Verfahren nach einem der vorangehenden Ansprüche, dadurch gekennzeichnet, dass das Alkaliiodid in einer Menge von 0,2 bis 50 mol je Grammatom Nickel verwendet wird.

13. Verfahren nach Anspruch 12, dadurch gekennzeichnet, dass das Alkaliiodid in einer Menge von 0,5 bis 20, vorzugsweise von 1 bis 10 mol je Grammatom Nickel verwendet wird.

14. Verfahren nach einem der vorangehenden Ansprüche, dadurch gekennzeichnet, dass der Kronenether mindestens 5 Mol-% des Alkaliiodids ausmacht.

15. Verfahren nach einem der vorangehenden Ansprüche, dadurch gekennzeichnet, dass der Kronenether höchstens 1 mol je Mol Alkaliiodid ausmacht.

16. Verfahren nach einem der vorangehenden Ansprüche, dadurch gekennzeichnet, dass der Kronenether 10 bis 50 Mol-% des Alkaliiodids ausmacht.

17. Verfahren nach einem der vorangehenden Ansprüche, dadurch gekennzeichnet, dass man ebenfalls in Gegenwart eines Lithiumsalzes arbeitet.

18. Verfahren nach Anspruch 16, dadurch gekennzeichnet, dass das Lithiumsalz ein Carboxylat mit maximal 12 Kohlenstoffatomen ist.

19. Verfahren nach einem der Ansprüche 17 oder 18, dadurch gekennzeichnet, dass das Lithiumsalz das Lithiumacetat ist.

## Claims

1. Process for preparing acetic anhydride by reaction of carbon monoxide with methyl acetate in an anhydrous medium, in liquid phase, at a total pressure of between 15 and 700 bar and at a temperature of between 140 and 300°C, in the presence of an effective quantity of nickel and of methyl iodide, characterised in that the operation is also carried out in simultaneous presence of an alkali metal iodide and a crown-ether.

2. Process according to Claim 1, characterised in that the total pressure is between 25 and 200 bar.

3. Process according to Claim 1 or 2, characterised in that the temperature is between 160 and 220°C.

4. Process according to one of the preceding claims, characterised in that the concentration of nickel is between 5 and 2,000 milligram atoms per litre of reaction medium.

5. Process according to Claim 4, characterised in that the concentration of nickel is between 20 and 1,000 milligram atoms per litre of reaction medium.

6. Process according to one of the preceding claims, characterised in that the crown-ether is chosen from the macrocyclic polyethers of Formulae(I) to(III):

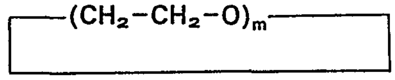

(I)

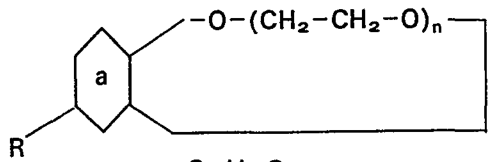

(II)

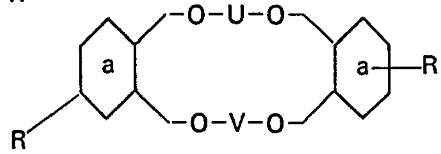

(III)

in which:

m and n are integers such that the macro-rings contain from 3 to 20 oxygen atoms,

a denotes a phenylene, cyclohexylene or 2,3-naphthylene radical,

R denotes an alkyl radical containing at most 4 carbon or hydrogen atoms,

U and V, which are identical or different, denote a radical of Formula(IV):

$$-(CH_2-CH_2-O)_{\overline{p}}(CH_2)_{\overline{q}} \qquad (IV)$$

in which p is an integer which may be zero and such that the ring contains at most 20 oxygen atoms and q is an integer between 1 and 10, q being preferably equal to 2 when p is other than zero.

7. Process according to Claim 6 characterised in that the crown-ether contains from 5 to 10 oxygen atoms in the ring.

8. Process according to one of the preceding claims, characterised in that the crown-ether is chosen from the compounds of Formula (III):

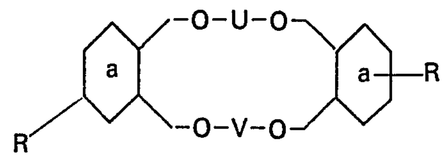

(III)

in which:

a denotes a phenylene, cyclohexylene or 2,3-naphthylene radical,

R denotes hydrogen,

U and V, which are identical, denote a radical of formula:

$$-(CH_2-CH_2-O)_{\overline{p}}(CH_2)_2$$

in which p is equal to 1, 2 or 3.

9. Process according to Claim 1, characterised in that the crown-ether is chosen from 15-crow-5-ether and 18-crown-6-ether.

10. Process according to one of the preceding claims, characterised in that methyl iodide is present in the reaction mixture at a concentration of 1 to 100 mol and, preferably, from 5 to 50 mol per gram atom of nickel.

11. Process according to one of the preceding claims, characterised in that the alkali metal iodide is chosen from sodium iodide and potassium iodide.

12. Process according to one of the preceding claims, characterised in that the alkali metal iodide

is used at a concentration of 0.2 to 50 mol per gram atom of nickel.

13. Process according to Claim 12, characterised in that the alkali metal iodide is used at a concentration of 0.5 to 20 and, preferably, from 1 to 10 mol per gram atom of nickel.

14. Process according to one of the preceding claims, characterised in that the crown-ether represents at least 5% (molar) of the alkali metal iodide.

15. Process according to one of the preceding claims, characterised in that the crown-ether represents at most 1 mol per mole of alkali metal iodide.

16. Process according to one of the preceding claims, characterised in that the crown-ether represents from 10 to 50% (molar) of the alkali metal iodide.

17. Process according to one of the preceding claims, characterised in that the operation is also carried out in the presence of a lithium salt.

18. Process according to Claim 16, characterised in that the lithium salt is a carboxylate containing at most 12 carbon atoms.

19. Process according to Claim 17 or 18, characterised in that the lithium salt is lithium acetate.